# FASCICULE DE BREVET EUROPEEN

(11) **EP 1 010 708 B1**
(45) Date de publication et mention de la délivrance du brevet: **31.03.2004**
(21) Numéro de dépôt: 99403051.8
(22) Date de dépôt: 07.12.1999
(51) Int. Cl.: C08F 2/32, A61K 7/48

(54) **Utilisation en cosmétique d'un latex inverse stable aux électrolytes**
Verwendung in der Kosmetik eines, gegen Elektrolyten, stabilen inversen Latex
Use in cosmetics of an inverse latex stable against electrolytes

(30) Priorité: 18.12.1998 FR 9816039; 09.02.1999 FR 9901497
(43) Date de publication de la demande: 21.06.2000
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Mallo, Paul, 78400 Chatou (FR); Tabacchi, Guy, 81100 Castres (FR); Boiteux, Jean-Pierre, 81710 Saix (FR)
(74) Mandataire: Conan, Philippe Claude

(56) Documents cités:
- EP-A- 0 186 361
- EP-A- 0 503 853
- FR-A- 2 710 263
- US-A- 5 185 395

## Description

La présente demande concerne des latex eau dans huile épaississants, leur procédé de préparation et leur application en tant qu'épaississant et/ou émulsionnant pour des produits de soins de la peau et des cheveux ou pour la fabrication de préparations cosmétiques, dermopharmaceutiques ou pharmaceutiques.

Différents épaississants existent et sont déjà utilisés pour ces usages. On connaît en particulier les produits naturels tels que les gommes de guar ou l'amidon mais dont les inconvénients sont ceux inhérents aux produits naturels, tels que la fluctuation des cours, les difficultés d'approvisionnement et une qualité aléatoire.

Les polymères synthétiques sous forme de poudre, principalement les polyacides acryliques sont également largement utilisés mais présentent l'inconvénient de nécessiter une neutralisation lors de l'utilisation car ils ne développent leur viscosité qu'à partir d'un pH supérieur à 6.5 et leur mise en solution est souvent fastidieuse.

Il existe aussi des polymères épaississants synthétiques, se présentant sous forme de latex inverses, c'est-à-dire dont la phase continue est une huile. La mise en solution de ces latex est extrêmement rapide ; les polymères contenus dans ces latex inverses sont le plus souvent des copolymères acrylamide / acrylate de métal alcalin ou acrylamide / acrylamido 2-méthyl 2-propanesulfonate de sodium ; ils sont déjà neutralisés et, lorsqu'ils sont mis en solution dans l'eau, par exemple à une concentration de 1 %, on observe que le pH est généralement supérieur à 6.
Les copolymères acrylamide / acrylate de sodium ne développent cependant pas de propriétés épaississantes importantes lorsqu'on abaisse le pH en dessous de 6 ; par contre les copolymères acrylamide/acrylamido 2-méthyl 2-propanesulfonate de sodium décrits dans EP 0 503 853, gardent une capacité épaississante importante même à pH égal à 4.

Cependant, de tels copolymères présentent des teneurs en monoacrylamide qui, bien qu'extrêmement faibles, pourraient conduire dans le futur, à rendre leur utilisation en cosmétique impossible, du fait de l'évolution de la réglementation européenne sur les substances dangereuses.

De plus, l'ensemble des polymères cités a tendance à perdre leur propriété épaississante, lorsque le milieu à épaissir, par exemple un produit cosmétique, contient des sels ; cette tendance s'accentue avec l'augmentation de la concentration en sels présents dans ledit milieu.

La demanderesse s'est donc intéressée à la recherche de nouvelles émulsions inverses sans acrylamide, qui soient plus stables aux électrolytes.

Selon un premier aspect de la présente invention, celle-ci a pour objet l'utilisation d'une composition comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 70% en poids, et de préférence de 25% à 40% en poids, d'un polyélectrolyte anionique, ledit polyélectrolyte anionique étant à base d'acide acrylique partiellement neutralisé, éventuellement branché et/ou réticulé, pour préparer une composition cosmétique, dermopharmaceutique ou pharmaceutique.

Par "agent émulsifiant du type eau dans huile", on désigne des agents émulsifiants possédant une valeur HLB suffisamment faible pour fournir des émulsions eau dans huile tels que les polymères tensioactifs commercialisés sous le nom de HYPERMER™ ou tels que les esters de sorbitan, comme le monooléate de sorbitan commercialisé par la Société SEPPIC sous le nom Montane™ 80 ou l'isostéarate de sorbitan commercialisé par SEPPIC sous le nom Montane™ 70.

Par "agent émulsifiant du type huile dans eau", on désigne des agents émulsifiants possédant une valeur HLB suffisamment élevée pour fournir des émulsions huile dans l'eau tels que, par exemple, les esters de sorbitan éthoxylés comme l'oléate de sorbitan éthoxylé à 20 moles d'oxyde d'éthylène, ou le laurate de sorbitan éthoxylé à 20 moles d'oxyde d'éthylène commercialisés par la société SEPPIC respectivement sous les noms MONTANOX™ 80 et MONTANOX™ 20 ou bien l'huile de ricin éthoxylée à 40 moles d'oxyde d'éthylène ou encore, l'alcool laurique éthoxylé à 7 moles d'oxyde d'éthylène commercialisé par la société SEPPIC respectivement sous les noms SIMULSOL™ OL 50 et SIMULSOL™ P 7.

Par acide acrylique partiellement neutralisé, on désigne plus particulièrement l'acide acrylique partiellement salifié sous forme de sel alcalin tel que le sel de sodium ou de potassium ou de sel de base azotée tel que le sel d'ammonium ou un sel avec un composé à ammonium quaternaire, tel qu'un sel d'aminoalcool. Il s'agit de préférence, de l'acide acrylique partiellement neutralisé sous forme du sel d'ammonium (NH₄⁺) ou de sel de monoéthanolamine (HOCH₂CH₂NH₃⁺).

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

Par aminoalcool, on entend les mono ou poly (hydroxyalkyl) amines

L'invention a plus particulièrement pour objet l'utilisation telle que définie précédemment, d'une composition, dans laquelle le polyélectrolyte anionique, est réticulé et/ou branché par un agent de réticulation et/ou un agent ramification choisi parmi, le triméthylolpropanetriacrylate, le diméthacrylate d'éthylèneglycol ou le méthylène bis(acrylamide) ou les composés comportant au moins deux radicaux allyle tel que par exemple l'acide diallyloxyacétique ou un de ses sels comme le diallyloxyacétate de sodium, le triallylamine, le diallylurée.

L'agent de réticulation et/ou de ramification est généralement utilisé dans la proportion molaire exprimée par rapport aux monomères mis en oeuvre, de 0,05% à 0,5%, et de préférence de 0,1% à 0,25%.

Le latex selon l'invention contient généralement de 2,5% à 15% en poids, et de préférence de 4% à 9% en poids, d'agents émulsifiants, parmi lesquels de 20% à 50%, notamment de 25% à 40% du poids total des agents émulsifiants présents sont du type eau dans huile (E/H) et dans laquelle de 80% à 50%, notamment de 75% à 60%, du poids total des agents émulsifiants, sont du type huile dans eau (H/E).

Selon un aspect particulier de la présente invention, la phase huile de la composition mise en oeuvre, représente de 15% à 40%, de préférence de 20% à 25%, du poids total de cette composition.

Cette phase huile est constituée soit par une huile minérale commerciale contenant des hydrocarbures saturés de type paraffine, isoparaffine, cycloparaffine, présentant à température ambiante, une densité entre 0.7 et 0.9 et un point d'ébullition supérieur à 180°C, telle que par exemple l'EXXOL™ D 100 S commercialisé par EXXON ou une huile blanche minérale telle que le MARCOL™ 52 également commercialisée par EXXON, l'isohexadécane commercialisé par BAYER ou l'isododécane, soit par une huile végétale comme l'hexaméthyltétracosane, ou squalane, soit par une huile de synthèse comme le polyisobutène ou le polyisobutène hydrogéné, soit par un mélange de plusieurs de ces huiles.

L'isohexadécane, qui est identifié dans Chemical Abstracts par le numéro RN = 93685-80-4, est un mélange d'isoparaffines en C₁₂, C₁₆ et C₂₀ contenant au moins 97% d'isoparaffines en C₁₆, parmi lesquelles le constituant principal est le 2,2,4,4,6,8,8-heptaméthyl nonane (RN = 4390-04-9). Il est commercialisé en France par la société Bayer. Le MARCOL™ 52 est une huile commerciale répondant à la définition des huiles de vaseline dans le Codex français. C'est une huile blanche minérale conforme aux règles FDA 21 CFR 178.878 et CFR 178.3620(a) ; elle est inscrite à la Pharmacopée des USA, US XXIII (1995) et à la Pharmacopée européenne (1993).

Selon un aspect préféré de la présente invention, la phase huile de la composition mise en oeuvre, comprend de l'isohexadécane ou une huile blanche minérale.

Les latex contiennent entre 20 % et 50 % d'eau. Ils peuvent également contenir divers additifs tels que des agents complexant, des agents de transfert ou des agents limiteurs de chaîne.

L'invention a aussi pour objet, une composition cosmétique, dermopharmaceutique ou pharmaceutique, caractérisée en ce qu'elle comprend au moins un latex inverse tel que défini précédemment ainsi qu'un procédé de préparation d'une composition cosmétique, dermopharmaceutique ou pharmaceutique caractérisé en ce que l'on y incorpore de 0,1% à 10% en poids d'une composition dont l'utilisation est objet de la présente invention.

La composition cosmétique, dermopharmaceutique ou pharmaceutique définie ci-dessus comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après shampooings. Il peut aussi être utilisé en combinaison avec lesdits SEPIGEL II est notamment compatible avec les concentrés décrits et revendiqués dans les publications internationales WO 92/06778, WO 95/04592, W095/13863, WO 98/47610 ou FR 2734 496 ou avec les agents tensioactifs décrits dans WO 93/08204.

Il est également compatible avec le MONTANOV™ 68, le MONTANOV™ 82, le MONTANOV™ 202 ou le SEPIPERL™ N.

Il peut être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024, ou encore en association avec des esters d'acides gras et de sucre, pour former des compositions pour le traitement du cheveu ou de la peau telles que celles décrites dans EP 0 603 019. ou encore dans les shampooings ou après shampooings tels que décrits et revendiqués dans WO 92/21316 ou enfin en association avec un homopolymère anionique tels que le CARBOPOL™ ou le PEMULEN™, pour former des produits de traitement des cheveux comme ceux décrits dans DE 195 23596.

Il est également compatible avec de nombreux principes actifs, tels que par exemple, les agents autobronzants comme le dihydroxyacétone (DHA) ou les agents anti-acné ; il peut donc être introduit dans des compositions autobronzantes comme celles revendiquées dans EP 0 715 845, EP 0604249, EP 0576188 ou dans WO 93/07902.

Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611.

Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

La composition selon l'invention est de façon toute particulière, compatible avec les actifs cosmétiques riches en sel minéraux, par exemple en sel de sodium ou de magnésium. Elle est compatible, notamment avec le SEPICALM™ S, le SEPICONTROL™ A5, le 2-pyrolidone carboxylate de sodium, le PROTEOL™ OAT, l'AJIDEW™ A100 ou le pyrolidone.

L'invention a enfin pour objet une composition cosmétique, dermopharmaceutique ou pharmaceutique telle que définie précédemment et contenant jusqu'à 5% en poids d'un ou plusieurs cations métalliques choisis notamment parmi les cations sodium, potassium, magnésium, manganèse ou aluminium.

Les exemples qui suivent, ont pour but d'illustrer la présente invention.

### Exemple 1 : Préparations de latex inverses

### Exemple 1a).

### Mode opératoire

On charge dans un bécher sous agitation :
- 250 g d'eau permutée,
- 250 g d'acide acrylique glacial,
- une solution à 30% d'ammoniaque de manière à amener le pH de la solution à 5,5, soit environ 170g,
- 0,45 g d'une solution commerciale à 40% de diéthylènetriamine pentaacétate de sodium,
- 2,31 g d'une solution commerciale à 50% de diallyloxyacétate de sodium,
- on complète avec de l'eau permutée jusqu'à 682 g.

Parallèlement, on prépare une phase organique en introduisant dans un bécher successivement et sous agitation :
- 220g d'isohexadécane,
- 30g de MONTANE™ 80 VG (oléate de sorbitan commercialisé par la société SEPPIC),
- 0,2g d'AIBN.

La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation. L'émulsion est soumise à un barbotage d'azote important, pour éliminer l'oxygène, et est refroidie à 5-6°C.

On introduit alors 0,16 g de peroxydisulfate de sodium dilué dans 20g d'eau.

Après un temps suffisant pour une bonne homogénéisation de la solution, on introduit alors, pendant 60 minutes, une solution aqueuse de métabisulfite de sodium (0,2g/100 ml d'eau) à raison de 0,5ml /minute.

Le milieu réactionnel est maintenu à cette température pendant 90 minutes.

L'ensemble est alors refroidi jusqu'environ 35°C puis on ajoute 50g de SIMULSOL™ OL50 pour obtenir l'émulsion désirée.

Evaluation des propriétés du latex obtenu
Viscosité à 25°C du latex à 3% dans l'eau (Brookfield RVT, Mobile 6, vitesse 20) : η = 30500 mPas;
Viscosité à 25°C du latex à 3% dans l'eau (Brookfield RVT, Mobile 6, vitesse 5) : η = 93000 mPas ;
Viscosité à 25°C du latex à 3% dans l'eau + 0,1% NaCI (Brookfield RVT, Mobile 6, vitesse 5) : η = 20200 mPas ;
Viscosité à 25°C du latex à 3% dans l'eau + 0,1% NaCI (Brookfield RVT, Mobile 6, vitesse 20) : η = 7600 mPas ;

### Exemple 1b)

En procédant comme à l'exemple 1a), mais en introduisant 50g de d'alcool laurique éthoxylé à 7 moles d'oxyde d'éthylène, au lieu du SIMULSOL™ OL 50, on obtient un latex ayant des propriétés viscosimétriques équivalentes.

### Exemple 1c)

En procédant comme à l'exemple 1a), mais en utilisant 0,95g de triallylamine, à la place des 2,31 g de solution commerciale à 50% de diallyloxyacétate de sodium, on obtient un latex ayant des propriétés viscosimétriques suivantes :
Viscosité à 25°C du latex à 3% dans l'eau (Brookfield RVT, Mobile 6, vitesse 20) : η = 36 200 mPas ;
Viscosité à 25°C du latex à 3% dans l'eau (Brookfield RVT, Mobile 6, vitesse 5) : η = 108 000 mPas ;
Viscosité à 25°C du latex à 3% dans l'eau + 0,1% NaCI (Brookfield RVT, Mobile 6, vitesse 5) : η = 16000 mPas ;
Viscosité à 25°C du latex à 3% dans l'eau + 0,1 % NaCI (Brookfield RVT, Mobile 6, vitesse 20) : η = 6200 mPas ;

### Exemple 1d)

### Mode opératoire

On charge dans un bécher sous agitation :
- 250 g d'eau permutée,
- 250 g d'acide acrylique glacial,
- 149,6 g de monoéthanolamine, de manière à amener le pH de la solution à 5,5 ;
- 0,45 g d'une solution commerciale à 40% de diéthylènetriamine pentaacétate de sodium,
- 3,4 g d'une solution commerciale à 50% de diallyloxyacétate de sodium,
- on complète avec de l'eau permutée jusqu'à 682 g.

Parallèlement, on prépare une phase organique en introduisant dans un bécher successivement et sous agitation :
- 220g d'isohexadécane,
- 30g de MONTANE™ 80 VG (oléate de sorbitan commercialisé par la société SEPPIC),
- 0,2g d'AIBN.

La phase aqueuse est introduite progressivement dans la phase organique puis soumise à une agitation mécanique violente de type ULTRA TURRAX™, commercialisé par IKA. L'émulsion obtenue est alors transférée dans un réacteur de polymérisation. L'émulsion est soumise à un barbotage d'azote important, pour éliminer l'oxygène, et est refroidie à 5-6°C.

On introduit alors 0,54 g de peroxydisulfate de sodium dilué dans 20g d'eau.

Après un temps suffisant pour une bonne homogénéisation de la solution, on introduit alors, pendant 60 minutes, une solution aqueuse de métabisulfite de sodium (0,4 g/100 ml d'eau) à raison de 0,5ml /minute.

Le milieu réactionnel est maintenu à cette température pendant 90 minutes.

L'ensemble est alors refroidi jusqu'environ 35°C puis on ajoute 50g de Laureth 7 pour obtenir l'émulsion désirée.

### Evaluation des propriétés du latex obtenu

Viscosité à 25°C du latex à 3% dans l'eau (Brookfield RVT, Mobile 6, vitesse 20) : η = 23 800 mPas;
Viscosité à 25°C du latex à 3% dans l'eau (Brookfield RVT, Mobile 6, vitesse 5) : η = 69 800 mPas ;
Viscosité à 25°C du latex à 3% dans l'eau + 0,1 % NaCI (Brookfield RVT, Mobile 6, vitesse 5) : η = 28 000 mPas ;
Viscosité à 25°C du latex à 3% dans l'eau + 0,1 % NaCI (Brookfield RVT, Mobile 6, vitesse 20) : η = 10 200 mPas ;

### Exemple 1e)

En procédant comme à l'exemple 1d), mais en utilisant 0,215 g de méthylène bis(acrylamide), à la place des 3,40 g de solution commerciale à 50% de diallyloxyacétate de sodium, on obtient un latex ayant des propriétés viscosimétriques suivantes :
Viscosité à 25°C du latex à 3% dans l'eau (Brookfield RVT, Mobile 6, vitesse 20) : η = 30 400 mPas ;
Viscosité à 25°C du latex à 3% dans l'eau (Brookfield RVT, Mobile 6, vitesse 5) : η = 92 000 mPas ;
Viscosité à 25°C du latex à 3% dans l'eau + 0,1% NaCI (Brookfield RVT, Mobile 6, vitesse 5) : η = 23 000 mPas ;
Viscosité à 25°C du latex à 3% dans l'eau + 0,1% NaCI (Brookfield RVT, Mobile 6, vitesse 20) : n = 8 600 mPas

### Exemple 1f)

En procédant comme à l'exemple 1d), mais en utilisant 0,039 g d'hydroperoxyde de cumène, à la place des 0,54 g de peroxydisulfate de sodium dilué dans 20g d'eau et 50 g de SIMULSOL™ OL 50 à la place des 50g de SIMULSOL™P7, on obtient un latex ayant des propriétés viscosimétriques suivantes :
Viscosité à 25°C du latex à 3% dans l'eau (Brookfield RVT, Mobile 6, vitesse 20) : η = 37 200 mPas ;
Viscosité à 25°C du latex à 3% dans l'eau (Brookfield RVT, Mobile 6, vitesse 5) : η = 114 000 mPas ;
Viscosité à 25°C du latex à 3% dans l'eau + 0,1% NaCI (Brookfield RVT, Mobile 6, vitesse 20) : η = 12 000 mPas ;
Viscosité à 25°C du latex à 3% dans l'eau + 0,1% NaCI (Brookfield RVT, Mobile 6, vitesse 5) : η = 35 000 mPas ;

Les exemples suivants mettent en oeuvre indifféremment l'une des compositions préparées aux exemples 1a à 1f (appelée composition 1).

### Exemple 2 : Crème de soin

| | |
|---|---|
| Cyclométhicone | 10% |
| Composition 1 | 0,8% |
| MONTANOV™ 68 | 2% |
| Alcool stéarylique | 1,5% |
| conservateur | 0,65% |
| Lysine | 0,025% |
| EDTA (sel disodique) | 0,05% |
| Gomme de xanthane | 0,2% |
| Glycérine | 3 % |
| Eau | q.s.p. 100% |

### Exemple 3 : Crème de soin

| | |
|---|---|
| Cyclométhicone | 10% |
| Composition 1 | 0,8% |
| MONTANOV™ 68 | 2% |
| Perfluoropolymethylisopropylether | 0,5% |
| Alcool stéarylique | 1,5% |
| conservateur | 0,65% |
| Lysine | 0,025% |
| EDTA (sel disodique) | 0,05% |
| PEMULEN™ TR1 | 0,2% |
| Glycérine | 3% |
| Eau | q.s.p. 100% |

### Exemple 4 : Baume après-rasage

| FORMULE | | |
|---|---|---|
| A | Composition 1 | 1,5% |
| | Eau | q.s.p 100% |
| B | MICROPEARL™ M 100 | 5,0% |
| | SEPICIDE™ CI | 0,50% |
| | Parfum | 0,20% |
| | Ethanol 95° | 10,0% |

### MODE OPERATOIRE

Ajouter B dans A.

### Exemple 5 : Emulsion satinée pour le corps

| FORMULE | | |
|---|---|---|
| A | SIMULSOL™ 165 | 5,0% |
| | LANOL™ 1688 | 8,50% |
| | Beurre de Karité | 2% |
| | Huile de paraffine | 6,5% |
| | LANOL™ 14M | 3% |
| | LANOL™ S | 0,6% |
| B | eau | 66,2% |
| C | MICROPEARL™ M 100 | 5% |
| D | Composition 1 | 3% |
| E | SEPICIDE™ CI | 0,3% |
| | SEPICIDE™ HB | 0,5% |
| | MONTEINE™ CA | 1% |
| | Parfum | 0,20% |
| | Acétate de vitamine E | 0,20% |
| | Sodium pyrolidinonecarboxylate | 1% (agent hydratant) |

### MODE OPERATOIRE

Ajouter C dans B, émulsionner B dans A à 70°C, puis ajouter D à 60°C puis E à 30°C

### Exemple 6 : Lait corporel

| FORMULE | | |
|---|---|---|
| A | SIMULSOL™ 165 | 5,0% |
| | LANOL™ 1688 | 12,0% |
| | LANOL™ 14M | 2,0% |
| | Alcool cétylique | 0,3% |
| | SCHERCEMOL™ OP | 3% |
| B | eau | q.s.p. 100% |
| C | Composition 1 | 0,35% |
| D | SEPICIDE™ CI | 0,2% |
| | SEPICIDE™ HB | 0,5% |
| | Parfum | 0,20% |

### MODE OPERATOIRE

Emulsionner B dans A vers 75°C ; ajouter C vers 60°C, puis D vers 30°C

### Exemple 7 : Crème H/E

| FORMULE | | |
|---|---|---|
| A | SIMULSOL™ 165 | 5,0% |
| | LANOL™ 1688 | 20,0% |
| | LANOL™ P | 1,0% (additif à effet stab |
| B | eau | q.s.p. 100% |
| C | Composition 1 | 2,50% |
| D | SEPICIDE™ CI | 0,20% |
| | SEPICIDE™ HB | 0,30% |

### MODE OPERATOIRE

Introduire B dans A vers 75°C ; ajouter C vers 60°C, puis D vers 45°C

### Exemple 8 : gel solaire non gras

| FORMULE | | |
|---|---|---|
| A | Composition 1 | 3,00% |
| | Eau | 30% |
| B | SEPICIDE™ CI | 0,20% |
| | SEPICIDE™ HB | 0,30% |
| | Parfum | 0,10% |
| C | colorant | q.s.p |
| | eau | 30% |
| D | MICROPEARL™ M 100 | 3,00% |
| | Eau | q.s.p 100% |
| E | huile de silicone | 2,0% |
| | PARSOL™ MCX | 5,00% |

### MODE OPERATOIRE

Introduire B dans A; ajouter C, puis D, puis E.

### Exemple 9 : Lait solaire

| FORMULE | | |
|---|---|---|
| A | SEPIPERL™ N | 3,0% |
| | Huile de sésame | 5,0% |
| | PARSOL™ MCX | 5,0% |
| | Carraghénane λ | 0,10% |
| B | eau | q.s.p.100% |
| C | Composition 1 | 0,80% |
| D | Parfum | q. s. |
| | Conservateur | a. s. |

### MODE OPERATOIRE

Emulsionner B dans A à 75°C puis ajouter C vers 60°C, puis D vers 30°C et ajuster le pH si nécessaire

### Exemple 10 : Gel de massage

| FORMULE | | |
|---|---|---|
| A | Composition 1 | 3,5% |
| | Eau | 20,0% |
| B | colorant | 2 gouttes/100g |
| | Eau | q. s. |
| C | alcool | 10% |
| | Menthol | 0,10% |
| D | huile de silicone | 5,0% |

### MODE OPERATOIRE

Ajouter B dans A ; puis ajouter au mélange, C puis D

### Exemple 11 : gel soin de massage

### FORMULE

| FORMULE | | |
|---|---|---|
| A | Composition 1 | 3,00% |
| | Eau | 30% |
| B | SEPICIDE™ CI | 0,20% |
| | SEPICIDE™ HB | 0,30% |
| | Parfum | 0,05% |
| C | colorant | q. s. |
| | Eau | q. s. p. 100% |
| D | MICROPEARL™ SQL | 5,00% |
| | LANOL™ 1688 | 2% |

### MODE OPERATOIRE

Préparer A ; additionner B, puis C, puis D.

### Exemple 12 : Gel coup d'éclat

| FORMULE | | |
|---|---|---|
| A | Composition 1 | 4% |
| | Eau | 30% |
| B | ELASTINE HPM | 5,0% |
| C | MlCROPEARL™ M 100 | 3% |
| | Eau | 5% |
| D | SEPICIDE™ CI | 0,2% |
| | SEPICIDE™ HB | 0,3% |
| | Parfum | 0,06% |
| Sodium pyrolidinonecarboxylate 50% | | 1% |
| Eau | | q. s. p. 100% |

### MODE OPERATOIRE

Préparer A ; additionner B, puis C, puis D.

### Exemple 13 : Lait corporel

| FORMULE | | |
|---|---|---|
| A | SEPIPERL™ N | 3,0% |
| | Triheptonate de glycérol | 10,0% |
| B | eau | q.s.p.100% |
| C | Composition 1 | 1,0% |
| D | parfum | q. s. |
| | Conservateur | q. s. |

### MODE OPERATOIRE

Fondre A à environ 75°C . Emulsionner B dans A à 75°C puis ajouter C vers 60°C, puis D .

### Exemple 14 : Emulsion démaquillante à l'huile d'amande douce

| FORMULE | |
|---|---|
| MONTANOV™ 68 | 5% |
| huile d'amande douce | 5% |
| eau | q.s.p.100% |
| Composition 1 | 0,3% |
| glycérine | 5% |
| conservateur | 0,2% |
| parfum | 03% |

### Exemple 15 : Crème hydratante pour peaux grasses

| FORMULE | |
|---|---|
| MONTANOV™ 68 | 5% |
| Cétylstéaryloctanoate | 8% |
| octyl palmitate | 2% |
| eau | q.s.p.100% |
| Composition 1 | 0,6% |
| MICROPEARL™ M100 | 3,0% |
| Mucopolysaccharides | 5% |
| SEPICIDE™ HB | 0,8 |
| Parfum | 0,3% |

### Exemple 16 : Baume après-rasage apaisant sans alcool

| FORMULE | |
|---|---|
| mélange de lauryl aminoacides | 0,1% à 5% |
| aspartate de magnésium et de potassium | 0,002% à 0,5% |
| LANOL™ 99 | 2% |
| huile d'amandes douces | 0,5% |
| eau | q.s.p.100% |
| Composition 1 | 3% |
| SEPICIDE™ HB | 0,3% |
| SEPICIDE™ CI | 0,2% |
| Parfum | 0,4% |

### Exemple 17 : Crème aux AHA pour peaux sensibles

| FORMULE | |
|---|---|
| mélange de lauryl aminoacides | 0,1% à 5% |
| aspartate de magnésium et de potassium | 0,002% à 0,5% |
| LANOL™ 99 | 2% |
| MONTANOV™ 68 | 5,0% |
| Eau | q.s.p.100% |
| Composition 1 | 1,50% |
| acide gluconique | 1,50% |
| tri éthylamine | 0,9% |
| SEPICIDE™ HB | 0,3% |
| SEPICIDE™ CI | 0,2% |
| Parfum | 0,4% |

### Exemple 18 : Soin apaisant après soleil

| FORMULE | |
|---|---|
| mélange de lauryl aminoacides | 0,1% à 5% |
| aspartate de magnésium et de potassium | 0,002% à 0,5% |
| LANOL™ 99 | 10,0% |
| Eau | q.s.p.100% |
| Composition 1 | 2,50% |
| SEPICIDE™ HB | 0,3% |
| SEPICIDE™ CI | 0,2% |
| Parfum | 0,4% |
| Colorant | 0,03% |

### Exemple 19 : Lait démaquillant

| FORMULE | |
|---|---|
| SEPIPERL™ N | 3% |
| PRIMOL™ 352 | 8,0% |
| huile d'amandes douces | 2 % |
| eau | q.s.p.100% |
| Composition 1 | 0,8% |
| conservateur | 0,2% |

### Exemple 20 : Lait corporel

| FORMULE | |
|---|---|
| SEPIPERL™ N | 3,5% |
| LANOL™ 37T | 8,0% |
| SOLAGUM™ L | 0,05% |
| Eau | q.s.p.100% |
| Benzophénone | 2,0% |
| diméthicone 350cPs | 0,05% |
| Composition 1 | 0,8% |
| conservateur | 0,2% |
| parfum | 0,4% |

### Exemple 21 : Fond de teint fluide

| FORMULE | |
|---|---|
| SIMULSOL™ 165 | 5,0% |
| LANOL™ 84D | 8,0% |
| LANOL™ 99 | 5,0% |
| Eau | q.s.p.100% |
| pigments et charges minérales | 10,0% |
| Composition 1 | 1,2% |
| conservateur | 0,2% |
| parfum | 0,4% |

### Exemple 22 : Lait solaire

| FORMULE | |
|---|---|
| SEPIPERL™ N | 3,5% |
| LANOL™ 37T | 10,0% |
| PARSOL NOX™ | 5,0% |
| EUSOLEX™ 4360 | 2,0% |
| Eau | q.s.p.100% |
| Composition 1 | 1,8% |
| conservateur | 0,2% |
| parfum | 0,4% |

### Exemple 23 : Gel contour des yeux

| FORMULE | |
|---|---|
| Composition 1 | 2,0% |
| Parfum | 0,06% |
| Sodium pyrrolidinonecarboxylate | 0,2% |
| DOW CORNING™ 245 Fluid | 2,0% |
| Eau | q. s. p. 100% |

### Exemple 24 : Composition de soin non rincée

| FORMULE | |
|---|---|
| Composition 1 | 1,5% |
| Parfum | q. s |
| Conservateur | q. s. |
| DOW CORNING™ X2 8360 | 5,0% |
| DOW CORNING™ Q2 1401 | 15,% |
| Eau | q.s.p. 100% |

### Exemple 25 : Gel amincissant

| | |
|---|---|
| Composition 1 | 5 % |
| Ethanol | 30 % |
| Menthol | 0,1 % |
| Caféine | 2,5 % |
| extrait de ruscus | 2 % |
| extrait de lierre | 2 % |
| SEPICIDE™ HP | 1 % |
| Eau | q. s. p. 100 % |

### Exemple 26 : Baume après-rasage apaisant sans alcool

| FORMULE | | |
|---|---|---|
| A | LIPACIDE™ PVB | 1,0% |
| | LANOL™ 99 | 2,0% |
| | Huile d'amandes douces | 0,5% |
| B | Composition 1 | 3,5% |
| C | eau | q.s.p.100% |
| D | parfum | 0,4% |
| | SEPICIDE™ HB | 0,4% |
| | SEPICIDE™ CI | 0,2% |

### Exemple 27 : Gel rafraîchissant après-rasage

| FORMULE | | |
|---|---|---|
| A | LIPACIDE™ PVB | 0,5% |
| | LANOL™ 99 | 5,0% |
| | Composition 1 | 2,5% |
| B | eau | q.s.p.100% |
| C | MICROPEARL™ LM | 0,5% |
| | Parfum | 0,2% |
| | SEPICIDE™ HB | 0,3% |
| | SEPICIDE™ CI | 0,2% |

### Exemple 28 : Soin pour les peaux grasses

| FORMULE | | |
|---|---|---|
| A | MICROPEARL™ M310 | 1,0% |
| | Composition 1 | 5,0% |
| | Isononanoate d'octyle | 4.0% |
| B | eau | q.s.p.100% |
| C | SEPICONTROL™ A5 | 4,0% |
| | Parfum | 0,1% |
| | SEPICIDE™ HB | 0,3% |
| | SEPICIDE™ CI | 0,2% |
| D | CAPIGEL™ 98 | 0,5% |
| | Eau | 10% |

### Exemple 29 : Crème aux AHA

| FORMULE | | |
|---|---|---|
| A | MONTANOV™ 68 | 5,0% |
| | LIPACIDE™ PVB | 1,05% |
| | LANOL™ 99 | 10,0% |
| B | eau | q.s.p.100% |
| | Acide gluconique | 1,5% |
| | TEA (triéthylamine) | 0,9% |
| C | Composition 1 | 1,5% |
| D | parfum | 0,4% |
| | SEPICIDE™ HB | 0,2% |
| | SEPICIDE™ Cl | 0,4% |

### Exemple 30 : Autobronzant non gras pour visage et corps

| FORMULE | | |
|---|---|---|
| A | LANOL™ 2681 | 3,0% |
| | Composition 1 | 2,5% |
| B | eau | q.s.p.100% |
| | Dihydroxyacétone | 3,0% |
| C | parfum | 0,2% |
| | SEPICIDE™ HB | 0,8% |
| | NaOH (hydroxyde de sodium) | qsp pH = 5 |

### Exemple 31 : Lait solaire au monoï de Tahiti

| FORMULE | | |
|---|---|---|
| A | Monoï de Tahiti | 10% |
| | LIPACIDE™ PVB | 0,5% |
| | Composition 1 | 2,2% |
| B | eau | q.s.p.100% |
| C | parfum | 0,1% |
| | SEPICIDE™ HB | 0,3% |
| | SEPICIDE™ Cl | 0,1% |
| | Méthoxycinnamate d'octyle | 4,0% |

### Exemple 32 : Soin solaire pour le visage

| FORMULE | | |
|---|---|---|
| A | Cyclométhicone et diméthiconol | 4,0% |
| | Composition 1 | 3,5% |
| B | eau | q.s.p.100% |
| C | parfum | 0,1% |
| | SEPICIDE™ HB | 0,3% |
| | SEPICIDE™ Cl | 0,21% |
| | Méthoxycinnamate d'octyle | 5,0% |
| | Micatitane | 2,0% |
| | Acide lactique | q.s.p. pH = 6,5 |

### Exemple 33 : Emulsion bronzante sans soleil

| FORMULE | | |
|---|---|---|
| A | LANOL™ 99 | 15% |
| | MONTANOV™ 68 | 5,0% |
| | Paraméthoxycinnamate d'octyle | 3,0% |
| B | eau | q.s.p.100% |
| | Dihydroxyacétone | 5,0% |
| | Phosphate monosodique | 0,2% |
| C | Composition 1 | 0,5% |
| D | parfum | 0,3% |
| | SEPICIDE™ HB | 0,8% |
| | NaOH | q.s.p pH=5. |

Les noms commerciaux cités ci-dessus, ont les définitions suivantes :
Le MONTANOV™ 68 (cétéaryl glucoside), est une composition auto-émulsionnable telle que décrite dans WO 92/06778, commercialisée par la société SEPPIC.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL® S sont des facteurs de consistance commercialisés par la société SEPPIC.
Le SEPICIDE™ HB , qui est un mélange de phénoxyéthanol, de méthyl paraben, d'éthylparaben, dé propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le PARSOL™ MCX est de l'octyl paraméthoxycinnamate; commercialisé par la société GIVAUDAN.
Le SEPIPERL™ N est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage; il est commercialisé par la société MATSUMO.
Le LANOL™ 99 est de l'isononyl isononanoate commercialisé par la société SEPPIC.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société ESSO.
Le LANOL™ 84D est du malate de dioctyle commercialisé par la société SEPPIC.
Le PARSOL NOX™ est un filtre solaire commercialisé par la société GIVAUDAN.
I' EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB,est un hydrolysat de protéines de blé palmitoylé, est commercialisée par la société SEPPIC.
Le MICROPEARL™ LM est un mélange de squalane, de poly(méthylméthacrylate) et de Menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Utilisation d'une composition comprenant une phase huile, une phase aqueuse, au moins un agent émulsifiant de type eau dans huile (E/H), au moins un agent émulsifiant de type huile dans eau (H/E), sous forme d'un latex inverse comprenant de 20% à 70% en poids, et de préférence de 25% à 40% en poids, d'un polyélectrolyte anionique, ledit polyélectrolyte anionique étant à base d'acide acrylique partiellement neutralisé, éventuellement branché et/ou réticulé, pour préparer une composition cosmétique, dermopharmaceutique ou pharmaceutique.

2. Utilisation telle que définie à la revendication 1, d'une composition, dans laquelle l'acide acrylique est partiellement salifié sous forme de sel alcalin tel que le sel de sodium ou de potassium, ou de sel de base azotée tel que le sel d'ammonium ou un sel avec un composé à ammonium quaternaire, tel qu'un sel d'aminoalcool.

3. Utilisation telle que définie à la revendication 2, d'une composition, dans laquelle l'acide acrylique est partiellement salifié sous forme du sel d'ammonium (NH₄⁺) ou de sel de monoéthanolamine (HOCH₂CH₂NH₃⁺).

4. Utilisation telle que définie à l'une des revendications 1 à 3, d'une composition, dans laquelle le polyélectrolyte anionique, est réticulé et/ou branché par un agent de réticulation et/ou un agent ramification choisi parmi, le triméthylolpropanetriacrylate, le diméthacrylate d'éthylèneglycol ou le méthylène bis(acrylamide) ou les composés comportant au moins deux radicaux allyle tel que par exemple l'acide diallyloxyacétique ou un de ses sels comme le diallyloxyacétate de sodium, le triallylamine, le diallylurée.

5. Utilisation telle que définie à l'une des revendications 1 à 4, d'une composition dans laquelle, la phase huile représente de 15% à 40% et de préférence de 20% à 25% de son poids total de cette composition.

6. Utilisation telle que définie à l'une des revendications 1 à 5, d'une composition dans laquelle la phase huile comprend de l'isohexadécane ou une huile blanche minérale.

7. Composition cosmétique, dermopharmaceutique ou pharmaceutique comprenant de 0,1% à 10% en poids d'un latex inverse tel que défini à l'une des revendications 1 à 6.

8. Composition cosmétique, dermopharmaceutique ou pharmaceutique telle que définie à la revendication 7, sous la forme d'un lait, d'une lotion, d'un gel, d'une crème, d'un savon, d'un bain moussant, d'un baume, d'un shampooing ou d'un après shampooing.

9. Composition cosmétique, dermopharmaceutique ou pharmaceutique telle que définie à l'une des revendications 7 ou 8 contenant jusqu'à 5% en poids, d'un ou plusieurs cations métalliques.

10. Composition cosmétique, dermopharmaceutique ou pharmaceutique telle que définie à la revendication 9, dont le ou les cations métalliques sont choisis parmi les cations sodium, potassium, magnésium, manganèse ou aluminium.

11. Procédé de préparation d'une composition cosmétique, dermopharmaceutique ou pharmaceutique, **caractérisé en ce que** l'on y incorpore de 0,1% à 10% en poids d'une composition telle que définie à l'une des revendications 1 à 6.

## Patentansprüche

1. Verwendung einer Zusammensetzung, enthaltend eine Ölphase, eine wäßrige Phase, mindestens einen Wasser-in-Öl-Emulgator (W/O-Emulgator) und mindestens einen Öl-in-Wasser-Emulgator (O/W-Emulgator), in Form eines inversen Latex. mit 20 bis 70 Gew.-% und vorzugsweise 25 bis 40 Gew.-% eines gegebenenfalls verzweigten und/oder vernetzten anionischen Polyelektrolyts auf Basis von teilneutralisierter Acrylsäure zur Herstellung einer kosmetischen, dermopharmazeutischen oder pharmazeutischen Zusammensetzung.

2. Verwendung nach Anspruch 1, bei der die Acrylsäure teilweise in Form eines Alkalisalzes, wie des Natrium- oder Kaliumsalzes, oder eines auf Stickstoff basierenden Salzes, wie des Ammoniumsalzes, oder eines Salzes mit einer quaternären Ammoniumverbindung, wie eines Aminoalkoholsalzes, vorliegt.

3. Verwendung nach Anspruch 2, bei der die Acrylsäure teilweise in Form des Ammoniumsalzes (NH₄⁺) oder des Monoethanolaminsalzes (HOCH₂CH₂NH₃⁺) vorliegt.

4. Verwendung nach einem der Ansprüche 1 bis 3, bei der der anionische Polyelektrolyt mit einem unter Trimethylolpropantriacrylat, Ethylenglykoldimethacrylat oder Methylenbis(acrylamid) oder Verbindungen mit mindestens zwei Allylresten, wie beispielsweise Diallyloxyessigsäure oder einem Salz davon, wie Natriumdiallyloxyacetat, Triallylamin oder Diallylharnstoff, ausgewählten Vernetzungs- und/oder Verzweigungsmittel vernetzt und/oder verzweigt ist.

5. Verwendung nach einem der Ansprüche 1 bis 4, bei der die Ölphase 15 bis 40 Gew.-% und vorzugsweise 20 bis 25 Gew.-% des Gesamtgewichts der Zusammensetzung ausmacht.

6. Verwendung nach einem der Ansprüche 1 bis 5, bei der die Ölphase Isohexadecan oder ein Weißöl enthält.

7. Kosmetische, dermopharmazeutische oder pharmazeutische Zusammensetzung, enthaltend 0,1 bis 10 Gew.-% eines inversen Latex nach einem der Ansprüche 1 bis 6.

8. Kosmetische, dermopharmazeutische oder pharmazeutische Zusammensetzung nach Anspruch 7 in Form einer Milch, einer Lotion, eines Gels, einer Creme, einer Seife, eines Schaumbads, eines Balsams, eines Shampoos oder einer Haarpflegespülung.

9. Kosmetische, dermopharmazeutische oder pharmazeutische Zusammensetzung nach Anspruch 7 oder 8 mit bis zu 5 Gew.-% eines oder mehrerer Metallkationen.

10. Kosmetische, dermopharmazeutische oder pharmazeutische Zusammensetzung nach Anspruch 9, in der das Metallkation bzw. die Metallkationen unter Natrium-, Kalium-, Magnesium-, Mangan- oder Aluminiumkationen ausgewählt ist bzw. sind.

11. Verfahren zur Herstellung einer kosmetischen, dermopharmazeutischen oder pharmazeutischen Zusammensetzung, **dadurch gekennzeichnet, daß** man darin 0,1 bis 10 Gew.-% einer Zusammensetzung nach einem der Ansprüche 1 bis 6 einarbeitet.

## Claims

1. Use of a composition comprising an oil phase, an aqueous phase, at least one emulsifier of water-in-oil (W/O) type and at least one emulsifier of oil-in-water (O/W) type, in the form of an inverse latex comprising from 20% to 70% by weight, and preferably from 25% to 40% by weight, of an anionic polyelectrolyte, the said anionic polyelectrolyte being based on partially neutralized acrylic acid, which may be branched and/or crosslinked, to prepare a cosmetic, dermopharmaceutical or pharmaceutical composition.

2. Use, as defined in Claim 1, of a composition in which the acrylic acid is partially salified in the form of an alkaline salt, such as the sodium or potassium salt, or in the form of the salt of a nitrogenous base, such as the ammonium salt, or a salt with a compound containing quaternary ammonium, such as an amino alcohol salt.

3. Use, as defined in Claim 2, of a composition in which the acrylic acid is partially salified in the form of the ammonium salt (NH₄⁺) or a monoethanolamine salt (HOCH₂CH₂NH₃⁺).

4. Use, as defined in one of Claims 1 to 3, of a composition in which the anionic polyelectrolyte is crosslinked and/or branched with a crosslinking agent and/or a branching agent chosen from trimethylolpropane triacrylate, ethylene glycol dimethacrylate or methylenebis(acrylamide) or compounds comprising at least two allyl radicals such as, for example, diallyloxyacetic acid or a salt thereof such as sodium diallyloxyacetate, triallylamine or diallylurea.

5. Use, as defined in one of Claims 1 to 4, of a composition in which the oil phase represents from 15% to 40% and preferably from 20% to 25% of its total weight of this composition.

6. Use, as defined in one of Claims 1 to 5, of a composition in which the oil phase comprises isohexadecane or a white mineral oil.

7. Cosmetic, dermopharmaceutical or pharmaceutical composition comprising from 0.1% to 10% by weight of an inverse latex as defined in one of Claims 1 to 6.

8. Cosmetic, dermopharmaceutical or pharmaceutical composition as defined in Claim 7, in the form of a milk, a lotion, a gel, a cream, a soap, a bubble bath, a balm, a shampoo or a conditioner.

9. Cosmetic, dermopharmaceutical or pharmaceutical composition as defined in either of Claims 7 and 8, containing up to 5% by weight of one or more metal cations.

10. Cosmetic, dermopharmaceutical or pharmaceutical composition as defined in Claim 9, in which the metal cation(s) is (are) chosen from sodium, potassium, magnesium, manganese and aluminium cations.

11. Process for preparing a cosmetic, dermopharmaceutical or pharmaceutical composition, **characterized in that** from 0.1% to 10% by weight of a composition as defined in one of Claims 1 to 6 is incorporated therein.
